# EUROPEAN PATENT SPECIFICATION

(11) **EP 0 347 225 B1**
(45) Date of publication and mention of the grant of the patent: **25.08.1993**
(21) Application number: 89306075.6
(22) Date of filing: 15.06.1989
(51) Int. Cl.: A61K 9/10, A61K 31/71

(54) **Pharmaceutical compositions containing primycin**
Primycin enthaltende Arzneimittelzusammensetzungen
Compositions pharmaceutiques contenant de la primycine

(30) Priority: 16.06.1988 HU 308888
(43) Date of publication of application: 20.12.1989
(73) Proprietor: CHINOIN Gyogyszer és Vegyészeti Termékek Gyára RT., H-1045 Budapest IV (HU)
(72) Inventor: Szabo, Anna Z., H-1163 Budapest (HU); Gaal, Joszef, H-1174 Budapest (HU); Marmarosi, Katalin, H-2051 Biatorbagy (HU); Sebestyen, Gyula, H-1089 Budapest (HU); Miholics, Gizella, H-1033 Budapest (HU); Kovacs, Marta, H-1108 Budapest (HU)
(74) Representative: Skailes, Humphrey John

(56) References cited:
- EP-A- 0 224 868
- DE-A- 2 514 873
- FR-A- 2 315 910
- GB-A- 2 103 085

## Description

The invention relates to a stable aqueous suspoemulsion containing primycin as active ingredient and its preparation. The invention also relates to pharmaceutical compositions prepared from the stable aqueous suspoemulsion.

It is known that primycin (18-arabinozyl-2-n-butyl-3,7,11,15,19,21,23,25,27,37-decahydroxy-4,16,32-34,36-pentamethyl-tetrakonta-16,32-dien-35-O-lacton-40-quanidiniumsulfate) can successfully be used for treating injuries of epithelium, injuries in the urology, surgery, ophthalmology, gynaecology, dermatology, and for household and burning injuries.

Neither resistant nor allergic reactions have been found in its use. Furthermore, combinations of primycin with other antibiotics are also known (HU-PS 158,241).

Primycin is used in different forms. HU-PS 173,708 describes pharmaceutical compositions containing heterocolloidal primycin. A disadvantage of these compositions is their low primycin content (0.2-1 %), and the ethanol used for preparing the heterocolloidal solvent has in certain cases a skin irritating effect. Also, if the alcohol evaporates from the treated surface, a part of the primycin taken up to the surface runs off without biological utilization.

HU-PS 194,493 describes a basic gel containing primycin and N-methyl-pyrralidone and the pharmaceutical compositions which can be prepared therefrom, such as gels and ointments.

An aim of the invention was to prepare a stable aqueous basic composition usable in itself having a high active ingredient content, which is alcohol-free, has a low organic solvent and surface active material content, and in addition is suitable for preparing many different forms of medicines.

The invention is an aqueous suspoemulsion containing primycin as active ingredient, which contains 0.2-5 weight% of primycin, 5-25 weight% of propyleneglycol, 0.5-5, weight% of non-ionic surface active agent, if desired up to 15 weight% of other auxiliary agent and distilled water in an amount necessary to 100 weight%.

The suspoemulsion of the invention can be prepared by dissolving or suspending warmly 0.2-5 weight% of primycin related to the end product in 5-25 weight% of propyleneglycol and mixing the obtained solution or suspension coldly or warmly with 0.5-5 weight% of non-ionic surface active agent, 0-15 weight% of other auxiliary agent, and then with water in an amount necessary to 100 mass%.

The suspoemulsion of the invention can also be used as such or by using known methods of the pharmaceutical production it can be transformed into compositions usable as pharmaceuticals or pharmaceutical cosmetics, such as gels, ointments, foam aerosols, bandages, or plasters.

In addition to primycin the compositions according to the invention can contain other known antimicrobial ingredients, such as sisomycin, nethylmycin, doxycyclin, gentamycin, norfloxacin, perfloxacin, cyprofloxacin, o-floxacin.

As non-ionic surface active agent, an ether of polyethyleneglycol formed with lauryl, cetyl, stearyl or oleylalcohol, sorbitan fatty acid ester or preferably ethoxylated stearyl alcohol can be used.

By using primycin having a particle size under 10 µm the active ingredient content of the compositions of the invention can be increased. The suspoemulsion is preferably used for preparing foam aerosols suitable for treating burns.

The characteristics of the foam compositions - a)loose, collapses quickly; b)loose but steady; b/hard - can be influenced by varying the concentration of the suitably choosen non-ionic surface active agent, preferably ethoxylated stearyl-alcohol (Polawax® A 31).

| The amount of Polawax A 31 | Foam density | Collapse time |
|---|---|---|
| 1 weight% | 0.26 g/cm³ | <60 minutes >3 hours |
| 1.5 weight% | 0.24 g/cm³ | <60 minutes >7 hours |
| 2.0 weight% | 0.22 g/cm³ | <60 minutes >12 hours |
| 2.5 weight% | 0.2 g/cm³ | >24 hours |

The hard foam prepared from the suspoemulsion according to the invention steadily adheres to the surface, covers the wound for a long time, ensures a bacterium-free environment and sometimes makes the use of bandage superfluous.

In the foam aerosol compositions Freon, propane-butane, isobutane or dinitrogenoxide are used as power gas.

The stability of the aqueous suspoemulsion of the invention was examined by microbiological evaluation and an organoleptic method.

The standard (Chinoin-Ebrimycin 841201 = 1066 mcg/mg) and the test material of Example 2 were dissolved in a measuring flask by 500 ml. of a mixture of butanol : ethanol : water in a ratio 1:1:2. The concentration of the thus-obtained strain solutions was 4 mcg/ml. from which the following diluting lines were prepared:

| Standard 4 mcg/ml. | Amount measured from the solution of test material according to the invention 4 mcg/ml |
|---|---|
| 0.80 ml | |
| 0.75 ml | 0.75 ml |
| 0.70 ml | 0.70 ml |
| 0.65 ml | 0.65 ml |
| 0.60 ml | 0.60 ml |
| 0.55 ml | 0.55 ml |

The test organism used was: Streptococcus ATCC 8043.
Culture medium: Standard Difco bouillon.
The amount of inoculeum was defined in a manner that the transmission should have been of 95 % compared to the (blind) culture medium without bacterium.

10 ml. of culture medium inoculated as described above were measured to each pre-prepared test tube.

Incubating temperature: 37 °C, incubation time: 3-5 hours.
Number of parallel measurements: 3

The measuring was carried out on spectrophotomether (Spektronom 195) at a wave-length of 570 nm by using a cuvet of 4 cm. The results were evaluated on the basis of the rules of mathematical statistics. On the basis of the microbiological comparative examinations it was found that the primycin content of the aqueous suspoemulsion according to the invention changed within a range of 5 %.

By examining the aqueous suspension according to the invention organolepticly, it can be stated that by storing at 37 °C the suspoemulsion does not suffer organoleptic change for 1 year.

From the aqueous suspoemulsion according to the invention the utilization of the active ingredient was tested by defining the MIC-value (minimal inhibiting concentration). As comparative material the current primycin containing Ebrymycin gel (HU-PS 173,708) was used.

Taking the actual primycin active ingredient content of the Ebrymycin gel determined by previous microbiological value determination and of the aqueous primycin suspoemulsion of 1 weight% into consideration, concentration series were prepared. The MIC determinations were carried out in accordance with the classic microbiological rules.
culture medium: Standard Difco bouillon
Incubating temperature: 37 °C
Incubation time: 24 hours
test organisms:
Staphylococcus aureus HNCMB 112002
Staphylococcus aureus HNCMB 112003
Staphylococcus aureus HNCM8 110002
Streptococcus faecalis HNCMB 80171
Streptococcus pyogenes HNCMB 80001
Escherichia coli HNCNB 35033
Pseudomonas aeruginosa HNCNB 170021
Number of parallel measurements: 3
The results of our examinations are summarized in Tables 1 to 6 as follows:

**Table 1**

| Streptococcus faecalis HNCMB 80171 | | | |
|---|---|---|---|
| Concentration of sample in the test tube (µg./ml.) | Ebrymycin gel MIC | Suspoemulsion according to | |
| | | Example 2 MIC | Example 12 MIC |
| 0.01 | + | + | + |
| 0.05 | + | + | + |
| 0.1 | + | + | - |
| 0.5 | + | - | - |
| 0.75 | + | - | - |
| 1.0 | + | - | - |
| 1.5 | + | - | - |
| 2.0 | + | - | - |
| 3.0 | + | - | - |
| 5.0 | - | - | - |
| 10.0 | - | - | - |

**Table 2**

| Streptococcus pyogenes HNCMB 80001 | | | |
|---|---|---|---|
| Concentration of sample in the test tube (µg./ml.) | Ebrymycin gel MIC | Suspoemulsion according to | |
| | | Example 2 MIC | Example 12 MIC |
| 0.01 | + | + | + |
| 0.05 | + | + | + |
| 0.1 | + | - | - |
| 0.5 | + | - | - |
| 0.75 | + | - | - |
| 1.0 | - | - | - |
| 1.5 | - | - | - |
| 2.0 | - | - | - |
| 3.0 | - | - | - |
| 5.0 | - | - | - |

**Table 3**

| Staphylococcus aureus HNCMB 110002 | | | |
|---|---|---|---|
| Concentration of sample in the test tube (µg./ml.) | Ebrymycin gel MIC | Suspoemulsion according to | |
| | | Example 2 MIC | Example 12 MIC |
| 0.01 | + | + | + |
| 0.05 | + | + | - |
| 0.1 | + | - | - |
| 0.5 | + | - | - |
| 0.75 | + | - | - |
| 1.0 | + | - | - |
| 1.5 | - | - | - |
| 2.0 | - | - | - |
| 3.0 | - | - | - |
| 5.0 | - | - | - |

**Table 4**

| Staphylococcus aureus HNCMB 112003 | | | |
|---|---|---|---|
| Concentration of sample in the test tube (µg./ml.) | Ebrymycin gel MIC | Suspoemulsion according to | |
| | | Example 2 MIC | Example 12 MIC |
| 0.01 | + | + | + |
| 0.05 | + | + | + |
| 0.1 | + | + | + |
| 0.5 | + | - | - |
| 0.75 | + | - | - |
| 1.0 | + | - | - |
| 1.5 | + | - | - |
| 2.0 | - | - | - |
| 3.0 | - | - | - |
| 5.0 | - | - | - |

**Table 5**

| Staphylococcus aureus HNCMB 112002 | | | |
|---|---|---|---|
| Concentration of sample in the test tube (µg./ml.) | Ebrymycin gel MIC | Suspoemulsion according to | |
| | | Example 2 MIC | Example 12 MIC |
| 0.01 | + | + | + |
| 0.05 | + | + | + |
| 0.1 | + | - | - |
| 0.5 | + | - | - |
| 0.75 | + | - | - |
| 1.0 | - | - | - |
| 2.0 | - | - | - |
| 3.0 | - | - | - |
| 5.0 | - | - | - |

**Table 6**

| Concentration of sample in the test tube (µg./ml.) | Escherichia coli HNCNB 35033 | Pseudomonas aeruginosa HNCNB 170021 |
|---|---|---|
| | Suspoemulsion according to Example 12 MIC | |
| 0.01 | + | + |
| 0.05 | + | + |
| 0.1 | + | - |
| 0.5 | + | - |
| 0.75 | + | - |
| 1.0 | - | - |
| 2.0 | - | - |
| 3.0 | - | - |
| 5.0 | - | - |

Evaluating the results, it can be stated that the aqueous suspoemulsion containing 1 weight% of primycin has about 10 times better biological utilization (on the pyogenic coccuses above tested) than Ebrymycin gel.

By well controlled comparative tests conducted under industrial conditions, it was to be clarified whether the composition is effective against clinical gargets. The effectivity was compared to the current Mamycin (Germed, GDR) veterinary composition of similar effectivity containing an ingredient with the same activity.

In a dairy farm the test comprised all the newly diagnostized cases showing the clinical symptoms of one type of acute gargets.

After samples were taken the medical treatment was carried out as follows: after the concerned udder quarters had been carefully molken out, the test composition was used intramammally (intracysternal). The content of a plastic syringe was injected in each case. The treatment was repeated similarly day after day for several days if the clinical status of the udder quarter so required. Before the medical treatment or by the control test, the cultured bacteria were determined from the milk samples gathered under aseptic conditions, while determining their total germ number, and the antibiotic sensitivity examination thereof was carried out.

When initiating the animals in the test, before beginning the medical treatment, the acute udder changes were put on the basis of the type of clinical symptoms among two main groups (catarrhal, parenchymal mastitis), within which several subgroups were distinguished on the basis of the seriousness of the symptoms. The classification was carried out on the basis of the points of view given by CSEH (1973), HORVÁTH (1983) and PYÖRALA (1983) adapted to the test conditions.

The change of the test conditions obtained as result of the medical treatment was examined daily. The final estimation was made daily on the basis of the control test carried out on the 10-22nd day after the last treatment (clinical, Mastitest, microbiological test).

Considering the points of view given by HORVÁTH (1982, 1983) the udder quarter was held to be recovered if no clinical symptoms or milk changes, in quantity or quality, relating to the inflammation were not found, and the result of the Mastitest examination of the secretion of glands and the microbiological test were negative. The case was considered as improved when the recovery had taken place with a little interstitial induration residue and by the permanent decrease of lower degree of the milk secretion. When, by control test, Mastitest positivity was found, it was noted as the decrease of the progress into subclinical, while in cases where the microbiological control test result was positive, as the silent infectedness of the udder quarter. In some cases, complete and definite drying up could be observed.

The tests comprised the 42 freshly sickened udder quarters of 35 animals. From this 32 udder quarters of 38 cows were catarrhal and 10 udder quarters of 7 animals proved to be parenchymal mastitis.

The classification of the test animals into groups is shown in Table 7.

About 2/3 of the test animals was subjected to a bacteriological test. When examining the catarrhal udder quarters having garget by microbiological methods before medical treatment, it was found that more than 1/3 proved to be bacteriologically negative.

In cases of galactephoromastitis of different seriousness each isolated bacteria was pathogenic of positive tincture (Str. or Staph.) according to Gram.

Each of the 32 udder quarters of the 28 animals with galactophoromastitis of different seriousness recovered (69 %) or at least became symptom-free (31 %). Generally 2 or 3 treatments were enough for the complete recovery (Table 8).

According to expectations in case of mastitis parenchymatosa acuta, the effectivity of the medical treatment was unfavourable: from the 10 sickened udder quarters of 7 animals, only 6 quarters recovered, 3 improved or lessened into a silent infection or subclinical case, while 1 molken out.

From the comparison (Table 8) it can be stated that the test treatment resulted a recovery to a large extent both in the case of catarrhal and paranchymal gargets.

**Table 7**

| Number of the treated and control groups | | | | |
|---|---|---|---|---|
| | Catarrhal mastitis | | Paranchymal mastitis | |
| | Cows | udder quarters | cows | udder quarters |
| Composition according to Example 12 | 28 | 20 | 4 | 6 |
| Mamycin | 10 | 12 | 3 | 4 |
| Total | 38 | 32 | 7 | 10 |

**Table 8**

| | Catarrhal udder quarters | | | Paranchymal mastitis udder quarters | | |
|---|---|---|---|---|---|---|
| | treated | recovered | improved | treated | recovered | improved |
| Composition according to Example 12 | 20 | 17 | 3 | 6 | 4 | 2 |
| | 100% | 85% | 15% | 100% | 67% | 33% |
| Mamycin | 12 | 7 | 5 | 4* | 2 | 1 |
| | 100% | 58% | 42% | 100% | 50% | 25% |
| | 32 | 22 | 10 | 10 | 6 | 3 |

| | | | | | | |
|---|---|---|---|---|---|---|
| * = one udder quarter molken out | | | | | | |
| Mamycin: (produced by Germed) active ingredient: benzyl-penicillin-K and streptomycin sulphate | | | | | | |

The compositions according to the invention are illustrated in the following non-limiting Examples. % are by weight.

### Example 1

Aqueous suspoemulsion with the following composition is prepared:

| | |
|---|---|
| Primycin | 0.2 % |
| Propyleneglycol | 15 % |
| Polawax® A 31 | 0.5 % |
| Distilled water ad | 100 % |

The pH of the composition is adjusted by Na₂HPO₂2H₂O to pH = 8.

### Example 2

Aqueous suspoemulsion with the following composition is prepared:

| | |
|---|---|
| Primycin | 1 % |
| Propyleneglycol | 15 % |
| Polawax® A 31 | 2.0 % |
| Distilled water ad | 100 % |

The pH of the composition is adjusted with Na₂HPO₄2H₂O to pH = 8.

### Example 3

Aqueous suspoemulsion with the following composition is prepared:

| | |
|---|---|
| Primycin | 1.5 % |
| Propyleneglycol | 25 % |
| Polawax® A 31 | 2.5 % |
| Distilled water ad | 100 %. |

The pH of the composition is adjusted with Na₂HPO₄2H₂O to pH = 8.

### Example 4

Aqueous suspoemulsion with the following composition is prepared:

| | |
|---|---|
| Microcrystalline primycin | 5 % |
| Propyleneglycol | 15 % |
| Polawax® A 31 | 3 % |
| Distilled water ad | 100 %. |

The pH of the composition is adjusted with Na₂HPO₄2H₂O to a value of 8.

### Example 5

The foam aerosol of the following composition is prepared:

| | |
|---|---|
| Composition of Example 4 | 85-90 % |
| Propellant (Freon 12/114, 50:50) | 10-15 %. |

### Example 6

A gel with the following composition is prepared:

| | |
|---|---|
| Suspoemulsion according to Example 4 | 50 % |
| Carbopol 934 | 2 % |
| Triethanolamine | 0.5 % |
| Conserving agent (Nipagin-M) | 0.1-0.2 % |
| Distilled water ad | 100 %. |

### Example 7

An ointment of the following composition is prepared:

| | |
|---|---|
| Suspoemulsion of Example 4 | 85 % |
| Cetyl-stearyl-alcohol | 10 % |
| Glycerine | 4.8-4.9 % |
| Conserving agent (Nipagin-M) | 0.1-0.12 %. |

### Example 8

A gauze sheet of the following composition is prepared:

| | |
|---|---|
| Suspoemulsion of Example 4 | 97.5 % |
| Polyvinyl-pyrrolidone | 0.5 % |
| Distilled water ad | 100 %. |

The composition is applied to a gauze sheet under sterile circumstances and covered.

### Example 9

The composition of Example 8 is applied to a sterile gauze sheet and, as cover sheet, thin, impregnated, shadow-proof linen with an adhesive surface is used.

### Example 10

Aqueous suspension of the following composition prepared:

| | |
|---|---|
| Primycin | 3 % |
| Gentamycin | 0.2 % |
| Propyleneglycol | 20 % |
| Polawax® A 31 | 3.5 % |
| Distilled water ad | 100 %. |

The pH of the composition is adjusted with Na₂HPO₄2H₂O to a value of 8.

### Example 11

A foam aerosol with the following composition is prepared:

| | |
|---|---|
| Composition of Example 4 | 80-85 % |
| Propellant (Propane-butane) | 15-20 %. |

### Example 12

Aqueous composition of the following composition is prepared:

| | |
|---|---|
| Primycin | 4 %. |
| Streptomycin sulphate | 2.6 % |
| Propyleneglycol | 16.0 % |
| Polawax® A 31 | 3.5 % |
| Prednisolone | 1.0 % |
| Distilled water ad | 100 %. |

## Claims

1. Stable aqueous suspoemulsion containing as active ingredient 0.2-5 weight% primycin, 5-25 weight% of propyleneglycol, 0.5-5 weight% of non-ionic surface active agent, if desired up to 15 weight% of auxiliary agent and distilled water in an amount necessary to 100 weight%.

2. Composition as claimed in claim 1 containing an ether of polyethyleneglycol formed with lauryl, cetyl, stearyl or oleyl alcohol; or sorbitan fatty acid ester or ethoxylated stearyl alcohol as non-ionic surface active agent.

3. Composition as claimed in claim 1 containing primycin of a particle size under 10 µm.

4. Antibacterial composition containing as active ingredient the primycin-containing stable aqueous suspoemulsion of claim 1 in an amount of 50-100 weight%, as well as filling, diluting and/or other forming auxiliary agents generally used in preparing pharmaceutical or pharmaceutical-cosmetic compositions, in an amount of 0-20 weight%.

5. Antibacterial composition according to claim 4 containing as active ingredient the primycin-containing stable aqueous suspoemulsion in an amount of 50-99.8 weight%, and a known antimicrobial active ingredient in an amount of 0.2-5 weight%.

6. A steady foam as claimed in claims 4 or 5 with regulated transmission of the active ingredient for treating wound surfaces, containing as active ingredient 0.2-4.5 weight% of primycin, 12.5-23 weight% of propyleneglycol, 0.8-2.3 weight% of non-ionic surface active agent, 0-2 weight% of known antimicrobial active ingredient, 10-20 weight% of foam-forming agent and distilled water in an amount necessary to 100 weight%.

7. Process for preparing a stable aqueous suspoemulsion of claims 1, 2 and 3 containing as active ingredient primycin, which comprises dissolving or suspending warmly 0.2-5 weight% of primycin, related to the end product, in 5-25 weight% of propyleneglycol, then mixing the obtained solution or suspension coldly or warmly with 0.5-5 weight% of non-ionic surface active agent, 0-15 weight% of other auxiliary agent, and then with water in an amount necessary to 100 weight%.

8. Process for preparing antibacterial composition of claims 4 or 5 which comprises transforming the aqueous stable suspoemulsion containing primycin as active ingredient into a pharmaceutical and/or pharmaceutical-cosmetic composition by using generally used filling, diluting and/or other formulating auxiliary agents and if desired, by adding other known antimicrobial active ingredient(s).

9. The process as claimed in claim 8 which comprises formulating the composition in form of gel, ointment, foam aerosol or other locally usable pharmaceutical form, as bandage or plaster.

## Patentansprüche

1. Stabile wäßrige Suspoemulsion, die enthält: 0,2 bis 5 Gew.-% Primycin als Wirkstoff, 5 bis 25 Gew.-% Propylenglycol, 0,5 bis 5 Gew.-% nichtionisches grenzflächenaktives Mittel, gewünschtenfalls bis zu 15 Gew.-% eines Hilfsstoffs, sowie destilliertes Wasser in einer auf 100 Gew.-% erforderlichen Menge.

2. Zusammensetzung nach Anspruch 1, die als nichtionisches grenzflächenaktives Mittel einen mit Laurylalkohol, Cetylalkohol, Stearylalkohol oder Oleoylalkohol gebildeten Polyethylenglycolether, Sorbitanfettsäureester oder ethoxylierten Stearylalkohol enthält.

3. Zusammensetzung nach Anspruch 1, die Primycin einer Teilchengröße von weniger als 10 µm enthält.

4. Antibakterielle Zusammensetzung, die als Wirkstoff die Primycin enthaltende stabile wäßrige Suspoemulsion nach Anspruch 1 in einer Menge von 50 bis 100 Gew.-% sowie Füllstoffe, Verdünnungsmittel und/oder andere Hilfsstoffe, die allgemein bei der Herstellung pharmazeutischer oder pharmazeutisch-kosmetischer Zusammensetzungen verwendet werden, in einer Menge von 0 bis 20 Gew.-% enthält.

5. Antibakterielle Zusammensetzung nach Anspruch 4, die als Wirkstoff die Primycin enthaltende stabile wäßrige Suspoemulsion in einer Menge von 50 bis 99,8 Gew.-% sowie einen bekannten antimikrobiellen Wirkstoff in einer Menge von 0,2 bis 5 Gew.-% enthält.

6. Beständige Schaumzusammensetzung nach Anspruch 4 oder 5 mit regulierter Freisetzung des Wirkstoffs zur Behandlung von Wundoberflächen, die als Wirkstoff 0,2 bis 4,5 Gew.-% Primycin, 12,5 bis 23 Gew.-% Propylenglycol, 0,8 bis 2,3 Gew.-% nichtionisches grenzflächenaktives Mittel, 0,2 bis 2 Gew.-% eines bekannten antimikrobiellen Wirkstoffs, 10 bis 20 Gew.-% eines schaumbildenden Mittels sowie destilliertes Wasser in einer auf 100 Gew.-% erforderlichen Menge enthält.

7. Verfahren zur Herstellung der stabilen wäßrigen Suspoemulsion nach den Ansprüchen 1 bis 3, die als Wirkstoff Primycin enthält, das folgende Schritte umfaßt: Lösen oder Suspendieren von 0,2 bis 5 Gew.-% Primycin, bezogen auf das Endprodukt, in 5 bis 25 Gew.-% Propylenglycol in der Wärme und anschließendes Mischen der erhaltenen Lösung oder Suspension in der Kälte oder in der Wärme mit 0,5 bis 5 Gew.-% nichtionischem grenzflächenaktivem Mittel und 0 bis 15 Gew.-% eines anderen Hilfsstoffs und dann mit Wasser in einer auf 100 Gew.-% erforderlichen Menge.

8. Verfahren zur Herstellung der antibakteriellen Zusammensetzung nach Anspruch 4 oder 5, wobei die wäßrige stabile Suspoemulsion, die als Wirkstoff Primycin enthält, unter Verwendung allgemein verwendeter Füllstoffe, Verdünnungsmittel und/oder anderer Formulierungshilfsstoffe sowie gewünschtenfalls unter Zusatz eines oder mehrerer anderer, bekannter antimikrobieller Wirkstoffe in eine pharmazeutische und/oder pharmazeutischkosmetische Zusammenstzung umgewandelt wird.

9. Verfahren nach Anspruch 8, wobei die Zusammensetzung in Form eines Gels, einer Salbe, eines Schaums, eines Aerosols oder einer anderen lokal anwendbaren pharmazeutischen Form, als Bandage oder als Pflaster formuliert wird.

## Revendications

1. Suspo-émulsion aqueuse stable contenant, à titre d'ingrédient actif, de 0,2 à 5% en poids de primycine, de 5 à 25% en poids de propylène-glycol, de 0,5 à 5% en poids d'un agent tensioactif non ionique, éventuellement jusqu'à 15% en poids d'un agent auxiliaire et de l'eau distillée en une quantité nécessaire q.s.p 100% en poids.

2. Composition selon la revendication 1, contenant un éther de polyéthylène-glycol formé avec l'alcool laurylique, cétylique, stéarylique ou oléylique ; ou un ester d'acide gras de sorbitanne ou un alcool stéarylique éthoxylé à titre d'agent tensioactif non ionique.

3. Composition selon la revendication 1, contenant de la primycine d'une granulométrie inférieure à 10 µm.

4. Composition antibactérienne contenant, à titre d'ingrédient actif, la suspo-émulsion aqueuse stable contenant de la primycine selon la revendication 1 à raison de 50 à 100% en poids, ainsi qu'une charge, des agents diluants et/ou autres agents auxiliaires qu'on utilise en général dans la préparation de compositions pharmaceutiques ou pharmaceutiques-cosmétiques, à raison de 0 à 20% en poids.

5. Composition antibactérienne selon la revendication 4, qui contient, à titre d'ingrédient actif, la suspo-émulsion aqueuse stable contenant la primycine en une proportion de 50 à 99,8% en poids et un ingrédient actif antimicrobien à raison de 0,2 à 5% en poids.

6. Mousse uniforme selon la revendication 4 ou 5, comprenant une transmission régulée de l'ingrédient actif afin de traiter les lésions superficielles, contenant, à titre d'ingrédient actif, de 0,2 à 4,5% en poids de primycine, de 12,5 à 23% en poids de propylène-glycol, de 0,8 à 2,3% en poids d'un agent tensioactif non ionique, de 0 à 2% en poids d'un ingrédient actif antimicrobien connu, de 10 à 20% en poids d'un agent porogène et de l'eau distillée q.s.p 100% en poids.

7. Procédé de préparation d'une suspo-émulsion aqueuse stable selon les revendications 1, 2 et 3, contenant, à titre d'ingrédient actif, de la primycine, qui consiste à dissoudre ou à mettre en suspension à chaud de 0,2 à 5% en poids de primycine, par rapport au produit final, dans 5 à 25% en poids de propylène-glycol, puis à mélanger la solution ou suspension obtenue à froid ou à chaud avec 0,5 à 5% en poids d'un agent tensioactif non ionique, 0 à 15% en poids d'un autre agent auxiliaire, puis avec de l'eau en une quantité nécessaire q.s.p 100% en poids.

8. Procédé de préparation d'une composition antibactérienne selon la revendication 4 ou 5, qui consiste à transformer la suspo-émulsion aqueuse stable contenant la primycine à titre d'ingrédient actif en une composition pharmaceutique et/ou pharmaceutique-cosmétique en utilisant une charge, un diluant et/ou d'autres agents auxiliaires de formulation qu'on utilise habituellement et, éventuellement, en ajoutant un ou plusieurs autre(s) ingrédient(s) antimicrobien(s) actif(s) connu(s).

9. Procédé selon la revendication 8, qui consiste à formuler la composition sous forme d'un gel, onguent, aérosol de mousse ou une autre forme pharmaceutique à usage local, en qualité de bandage ou de plâtre.
